# EUROPÄISCHE PATENTSCHRIFT

(11) **EP 0 915 166 B1**
(45) Veröffentlichungstag und Bekanntmachung des Hinweises auf die Patenterteilung: **11.02.2004**
(21) Anmeldenummer: 98120626.1
(22) Anmeldetag: 02.11.1998
(51) Int. Cl.: C12N 15/86, C12N 5/10, C12N 1/21

(54) **DNA-Virus-Vektoren und Verfahren zu ihrer Herstellung**
DNA virus vectors and methods for their production
Vecteurs de virus à ADN et leurs procédés de production

(30) Priorität: 05.11.1997 DE 19748895; 27.03.1998 DE 19813776
(43) Veröffentlichungstag der Anmeldung: 12.05.1999
(73) Patentinhaber: GSF-Forschungszentrum für Umwelt und Gesundheit GmbH, 85764 Oberschleissheim (DE)
(72) Erfinder: Delecluse, Henri-Jacques, 80335 München (DE); Pich, Dagmar, 80637 München (DE); Hammerschmidt, Wolfgang, 80686 München (DE)
(74) Vertreter: Reinhard - Skuhra - Weise & Partner

(56) Entgegenhaltungen:
- EP-A- 0 694 613
- WO-A-99/06582
- DE-A- 19 733 364
- KRISKY D. M. ET AL.: "Rapid method for construction of recombinant HSV gene transfer vectors" GENE THERAPY, Bd. 10, Nr. 4, Oktober 1997, Seite 1120 1120 XP002079298
- O'CONNOR M ET AL: "CONSTRUCTION OF LARGE DNA SEGMENTS IN ESCHERICHIA COLI" SCIENCE, Bd. 244, Nr. 4910, 16. Juni 1989, Seiten 1307-1312, XP000051939
- KEMPKES B. ET AL.: " Immortalization of human primary B lymphocytes in vitro with DNA." PROC. NATL. ACAD. SCI. USA, Bd. 92, Nr. 13, 1995, Seiten 5875-58789, XP002094030
- KEMPKES B. ET AL.: "Immortalization of human B lymphocytes by a plasmid containing 71 kilobas pairs of Epstein-Barr virus DNA." JOURNAL OF VIROLOGY, Bd. 69, Nr. 1, 1995, Seiten 231-238, XP002094031
- DELECLUSE H.-J. ET AL.: "Propagation and recovery of intact, infectious Epstein-Barr virus from prokaryotic to human cells." PROC. NATL. ACAD. SCI. USA, Bd. 95, Juli 1998, Seiten 8245-8250, XP002094032
- SAUER B. ET AL: 'Site specific insertion of DNA into a pseudorabies virus vector' PROC. NATL. ACAD. SCI. Bd. 84, 1987, USA, Seiten 9108 - 9112

## Beschreibung

Die vorliegende Erfindung betrifft ein Verfahren zur Herstellung von DNA-Virus-Vektoren, die sowohl in eukaryontischen als auch prokaryontischen Zellen replizieren.

### Stand der Technik

Epstein-Barr Virus (EBV) ist eines von verschiedenen Herpesviren des Menschen. Die DNA Sequenz des B95.8 Isolats von EBV ist bestimmt (Baer *et al*., 1984) und detaillierte wissenschaftliche Erkenntnisse sind vor allem über DNA-Elemente erarbeitet worden, die in den beiden Phasen des EBV maßgeblich beteiligt sind. In der sogenannten 'latenten Phase' geht das Virus eine stabile Wirtszellbeziehung ein, in der die Vitalität der Zelle nicht gestört wird, das virale DNA Genom aber als extrachromosomales Plasmid in den Wirtszellen repliziert und an die Tochterzellen weitergegeben wird. Die latente Phase kann mit der Transformation, bzw. Immortalisation der latent infizierten Zellen einhergehen. Der plasmidale Replikationsursprung oriP ist das DNA-Element, das für die Aufrechterhaltung und Replikation des EBV-Genoms in der latenten Phase essentiell ist(Yates *et al*., 1985). Dieses DNA-Element ist auch in rekombinanten Plasmiden aktiv und hat als solches verschiedene Anwendungen erfahren.

In der sogenannten 'lytischen oder produktiven Phase' wird Virus aktiv produziert. was neben der Expression von fast allen viralen Proteinen, die zur Regulierung der Expression oder zur Expression von viralen Strukturkomponenten nötig sind, auch zwei weitere DNA-Elemente des Virus involviert: der lytische Replikationsursprung, oriLyt, ist verantwortlich für die Amplifikation viraler DNA (Hammerschmidt und Sugden, 1988), die Terminal Repeats, TR, sind die Verpackungssignale, die für die Enkapsidierung der amplifizierten EBV DNA unabdingbar nötig sind (Hammerschmidt and Sugden, 1989).

Weitreichendes Interesse besteht sowohl an der genetischen Analyse der EBV Funktionen als auch an der Konstruktion von rekombinanten EBV Genomen. Das Interesse beruht darauf, daß EBV Genome zusätzliche, therapeutische Gene tragen können oder daß bestimmte unerwünschte Eigenschaften oder Gene des EBV entfernt werden. Die bisherigen Techniken, das EBV Genom zu verändern, beruhen auf homologen Rekombinationsereignissen von rekombinanten E. coli Plasmiden mit EBV Genomen in latent EBV infizierten Zellinien .(Cohen *et al*., 1989; Hammerschmidt and Sugden, 1989).. Alternativ können sog. 'mini-EBV-Genome' in E. coli hergestellt werden, die allerdings nur die viralen Genfunktionen tragen, die für die latente Phase von EBV wichtig sind .(Sugden und Hammerschmidt, 1993; Hammerschmidt und Sugden, 1995: Kempkes *et al*., 1995a; Kempkes *et al*., 1995b)..

PROC. NATL. ACAD. SCI. USA, vol. 84, December 1987, pages 9108-9112, B. Sauer et al., "Site specific insertion of DNA into a pseudorabies virus vector" offenbart DNA-Virus-Vektoren, die aus einer in vitro-Integration eines Plasmids in das Genom eines Alpha-Herpes-Virus mit einer Größe von 150kbp hervorgehen.

Die WO 99/06582 beansprucht die Priorität vom 01.08.1997. Der Inhalt in der ursprünglich eingereichten Fassung gilt gemäß Artikel 54 (3) und (4) EPÜ als bei der Prüfung auf Neuheit zu berücksichtigender Stand der Technik. Das Dokument beschreibt ein Verfahren zum Klonieren eines großen Virusgenoms, umfassend das Einbringen einer Sequenz, enthaltend Sequenzen eines Klonierungsvehikels, in eine Zelle, enthaltend das zu klonierende Virusgenom, und das Rekombinieren der zuerst genannten Sequenz mit dem zu klonierenden Virusgenom, vorzugsweise über homologe Rekombination, so daß ein künstliches zirkuläres Chromosom entsteht. Als Beispiel für die zu verwendende virale DNA werden Herpes-Viren genannt.

### Nachteile des Stands der Technik

Es ist bisher nicht möglich, gezielt und mit hoher Effizienz jedes beliebige Gen von DNA-Viren >=100 kbp, beispielsweise des EBV, zu verändern oder zu deletieren. So sind die homologen Rekombinationsereignisse zwischen einem in E. coli klonierten Abschnitt von EBV und dem endogenen EBV, das in latent infizierten Zellinien vorhanden ist, ungenau, schwer zu kontrollieren und müssen über kotransfizierte Markerabschnitte oder selektionierbare Gene sichtbar gemacht werden. Beim derzeitigen Stand der Technik sind diese Ansätze nicht universell anwendbar, langsam, und erlauben nicht, Gene oder genomische Abschnitte von DNA Viren >= 100 kbp von EBV zu verändern, die z.B. für die Aufrechterhaltung der latenten Phase des EBV-Virus in den infizierten Zellen notwendig sind.

### Aufgabenstellung

Es ist eine Aufgabe der vorliegenden Erfindung, ein Verfahren zur Herstellung von DNA-Virus-Vektoren, die sowohl in eukaryontischen als auch in prokaryontischen Zellen replizierbar sind, bereitzustellen, wodurch rekombinante DNA-Viren mit einem Genom >= 100 kbp herstellbar sind.

Diese Aufgabe wird durch das Verfahren gemäß Anspruch 1 gelöst. Bevorzugte Ausgestaltungen des erfindungsgemäßen Verfahrens ergeben sich aus den Unteransprüchen.

Erfindungsgemäß werden somit die Nachteile des Standes der Technik dadurch behoben, daß das gesamte DNA-Virus-Genom von Viren mit einer Größe >= 100 kbp als funktionsfähige Einheit kloniert wird. Die Klonierung in z.B. E. coli als rekombinantes Molekül setzt voraus, daß es gelingt, einen sogenannten prokaryonten Genabschnitt in das intakte DNA-Virus-Genom zu integrieren. Die Integration eines solchen Abschnitts, der Replikationsfunktionen und einen selektionierbaren Marker für E. coli trägt, stellt sicher, daß so ein rekombinantes Molekül in der Größe von mehr als 100 kbp in z.B. E. coli transferierbar ist und dort stabil repliziert. Dieser Vorgang stellt zugleich sicher, daß über herkömmliche rekombinante DNA Technologien die Modifikation aller DNA-Virus-Abschnitte, z.B. in E. coli, möglich ist.

Nachfolgend wird das erfindungsgemäße Verfahren am Beispiel eines Herpes Virus, nämlich von EBV, beschrieben..

Das beschriebene Verfahren erlaubt die Herstellung von rekombinanten Herpesvirusgenomen, die in E. coli klonal vermehrt werden können. Mit Sicherheit kann man davon ausgehen, daß dieses Verfahren auf alle großen DNA Viren angewandt werden kann (z. B. Poxviren, Iridioviren, andere Herpesviren, Baculoviren). Die Klonierung dieser Virusgenome in z.B. E. coli erlaubt die einfache Modifikation durch gezielte Veränderungen viraler Gene, ihre Deletion oder das Hinzufügen von weiteren Genen, die von Interesse sind. Die so hergestellten viralen Genome weisen je nach genetischer Modifikation neue Eigenschaften auf, die z. B. die Expression von Fremdproteinen in den jeweiligen Zielzellen der Viren erlauben.

Die in z.B. E. coli klonierten viralen Genome stellen ein Produkt dar, das sich zur Herstellung von Viruspartikeln einsetzen läßt, die z. B. für die Enkapsidierung von rekombinanten Plasmiden oder subgenomischen viralen Abschnitten geeignet sind.

Das erfindungsgemäße Verfahren dient zur Herstellung von DNA-Virus-Vektoren, die sowohl in eukaryontischen als auch prokaryontischen Zellen replizierbar sind und umfaßt zumindest die nachfolgenden Schritte:
a) Einbringen eines DNA-Virus-Genoms ≥ 100 kbp in eine eukaryontische Zielzelle und Etablieren solcher Zellen, die das virale DNA-Genom zumindest in extrachromosaler Form enthalten; oder Einsetzen einer einer DNA-Virus-Genom ≥ 100 kbp in extrachromosomaler Form bereits natürlicherweise enthaltenden eukaryontischen Zielzelle
b) Einbringen eines DNA-Abschnitts in die eukaryontische Zielzelle, der zumindest, in funktioneller Anordnung, die Information für die Replikation des viralen DNA-Genoms in prokaryontischen Zellen, ein in eukaryontischen Zellen selektierbares Markergen und zumindest ein in prokaryontischen Zellen selektierbares Markergen umfaßt, und der von DNA-Abschnitten (homologe Abschnitte) aus dem DNA-Virus-Genom in einer eine Rekombination ermöglichenden Länge flankiert ist;
c) Integration der unter (b) bezeichneten Abschnitte in das DNA-Virus-Genom durch Rekombination;
d) Selektion auf diejenigen Zielzellen, die ein extrachromosomales, rekombinantes, virales DNA-Genom enthalten; wahlweise
e) Aufreinigen und Isolieren des rekombinanten DNA-Virus-Vektor-Genoms.

Das erfindungsgemäße Verfahren unterscheidet sich von bisher bekannten Verfahren zur Herstellung von DNA-Virus-Vektoren insbesondere dadurch, daß zum ersten Mal die Herstellung derartiger Vektoren aus DNA-Viren mit einer Größe >= 100 kbp ermöglicht wird. Im bisherigen Verfahren wurden beispielsweise DNA-Viren wie Adeno-Viren mit einer Größe von ca. 40 kbp kloniert. Die Herstellung von DNA-Virus-Vektoren mit einer Größe von >= 100 kbp. insbesondere >= 120 kbp, bevorzugt >= 150 kbp oder insbesondere bevorzugt >= 170 kbp war mit den aus dem Stand der Technik bekannten Verfahren nicht möglich.

Durch das erfindungsgemäße Verfahren können nicht nur rekombinante Herpes-Virus-Genome hergestellt werden, die sowohl in prokaryonten Zellen als auch eukaryonten Zellen vermehrbar sind, sondern z.B. auch rekombinante Pox-Virus- oder Iridio-Virus- oder Baculo-Virus-Genome.

Das Herpes Virus kann ein Alpha-Herpes-Virus, ein Beta-Herpes-Virus oder ein Gamma-Herpes-Virus sein. Beispiele für diese Viren sind: Alpha-Herpes-Virus: Herpes-Simplex-Virus (HSV), Varicella-Zoster-Virus (VZV); Beta-Herpes-Virus: Cytomegalie-Virus (CMV); Gamma-Herpes-Virus: Epstein-Barr-Virus (EBV)

Das Einbringen des DNA-Virus in die eukaryontische Zielzelle kann durch an sich bekannte Methoden erfolgen; Beispiele hierfür sind Infektion, Transfektion oder Elektroporation. Gleiches gilt für das Einbringen des DNA-Abschnitts in die eukaryontische Zielzelle im Schritt (b).

Im Verfahrensschritt (a), bei dem das DNA-Virus in die eukaryontische Zielzelle eingeführt wird, können solche Zellen verwendet werden, die das DNA-Virus bereits in extrachromosomaler Form enthalten.

Als Zielzelle ist erfindungsgemäß jede Zelle zu verstehen, die Rezeptoren für das Virus besitzt und in denen das Virus replizieren kann.

Neben dem DNA-Virus wird weiterhin ein DNA-Abschnitt in die eukaryontische Zielzelle eingebracht, der mindestens die Information für die Replikation des viralen DNA-Genoms in prokaryontischen Zellen und ein in prokaryontischen Zellen selektierbares Marker-Gen umfaßt. Diese Genabschnitte werden von DNA-Abschnitten flankiert, die eine solche Länge aufweisen, daß eine Rekombination mit dem DNA-Virus ermöglicht wird. Die flankierenden DNA-Abschnitte weisen Homologien zum DNA-Virus auf, so daß eine Rekombination, bevorzugt eine homologe Rekombination, ermöglicht wird. Die Länge der flankierenden DNA-Sequenzen liegt bevorzugt bei >= 300 bp, weiterhin bevorzugt bei >= 1 kbp und insbesondere bevorzugt bei >= 2 kbp.

Die Integration der im Schritt (b) bezeichneten Abschnitte in das DNA-Virus-Genom erfolgt bevorzugt durch eine homologe Rekombination in der Zielzelle des DNA-Virus. Es ist aber auch möglich, daß eine illegitime Rekombination zum gewünschten Ergebnis führt. Normalerweise wird jedoch eine homologe Rekombination das rekombinierende DNA-Virus-Genom ermöglichen.

Der DNA-Abschnitt im Schritt (b) kann vor dem Einbringen in die eukaryontische Zielzelle linearisiert werden. Die Enden werden so gegen einen Abbau durch endogene Nukleasen geschützt.

In einer bevorzugten Ausführungsform der Erfindung befinden sich die Information für die Replikation des Virus-DNA-Genoms in Prokaryonten und die Information für das in prokaryontischen Zellen selektierbare Marker-Gen auf dem F-Plasmid von E. coli. In einer weiteren bevorzugten Ausführungsform der Erfindung enthält der DNA-Abschnitt im Schritt (b) zusätzlich zumindest ein in eukaryonten Zellen selektierbares Markergen. Als selektierbare Marker können beliebige, in prokaryonten Zellen bzw. in eukaryonten Zellen selektierbare Marker verwendet werden. Beispiele hierfür sind insbesondere Antibiotikaresistenzgene und Fluoreszenzmarkergene.

F-Faktor abgeleitete Plasmide mit ca. 5 kpb enthalten in der Regel folgende Abschnitte und Gene des E. coli F-Faktorplasmids, das in der natürlich vorkommenden Form um die 100 kpb groß ist: für die Aufrechterhaltung der Kopienzahl um 1 bis 2 Kopien/E. coli Zelle sind die Gene parA und parB wesentlich, für die DNA-Replikation werden oriS und das Gen repE benötigt. Alle diese Elemente befinden sich auf dem F-Faktor-Plasmid, das z.B. für die Generation des EBV/F-Faktor-Konstrukts verwandt wurde.

In einer weiteren Ausführungsform der Erfindung befindet sich auf dem DNA-Abschnitt im Verfahrensschritt (b) zumindest ein therapeutisch wertvolles Gen, das beispielsweise für ein Protein der Blutgerinnungskaskade codiert, beispielsweise für das Faktor VIII-Gen. Beliebige, an sich bekannte Gene können auf dem DNA-Abschnitt lokalisiert sein und durch das erfindungsgemäße Verfahren in den DNA-Virus-Shuttle-Vektor eingebracht werden. Hierdurch ist es möglich, das Gen von Interesse entweder in einer eukaryonten Zelle oder in einer prokaryonten Zelle zu exprimieren.

Durch das erfindungsgemäße Verfahren wird die Klonierung vollständiger DNA-Virus-Genome mit einer Größe >= 100 kbp ermöglicht. Ein besonders bevorzugtes Beispiel ist das EBV-Genom in einer Größe von ca. 170 kbp.

In einer Ausführungsform der vorliegenden Erfindung werden die homologen Abschnitte in (b) so gewählt, daß eine Mutation in das virale Genom eingeführt wird. Unter Mutation wird erfindungsgemäß eine Punktmutation, eine mehrere Nukleotide betreffende Mutation, eine Deletion, eine Addition oder ein Austausch von Nukleotiden verstanden. Die Addition von Nukleotiden umfaßt auch das Einführen von ein oder mehreren Genen, die ein therapeutisch wertvolles Gen, kodieren.

In einer Ausführungsform des erfindungsgemäßen Verfahrens werden die flankierenden Abschnitte in Verfahrensschritt (b) so ausgewählt, daß die terminalen Repeats im herpesviralen Genom deletiert werden. Nach Rekombination mit dem DNA-Virus-Genom entsteht ein verpackungsdefektes virales Genom, wodurch nach Induktion der lytischen Phase von EBV in den Zielzellen keine infektiösen Partikel freigesetzt werden, während gleichzeitig jedoch alle viralen Proteine und Funktionen zur Verpackung von DNA-Molekülen in trans zur Verfügung gestellt werden.

In einer weiteren Ausführungsform der Erfindung wird das erfindungsgemäß erhaltene rekombinante DNA-Virus-Vektor-Genom in weiteren Verfahrensschritten gemäß Anspruch 18 mutiert. Eine Definition, was erfindungsgemäß unter "Mutation" zu verstehen ist, wurde bereits weiter oben gegeben. Die Einführung der Mutationen kann sowohl in der oben beschriebenen Weise erfolgen, aber auch dadurch, daß das erhaltene DNA-Virus-Vektor-Genom in eine Prokaryontenzelle eingeführt wird oder eine andere Eukaryontenzelle und in diesen Zellen, beispielsweise durch weitere Rekombinationsereignisse, die Mutationen eingeführt werden. In einer bevorzugten Ausführungsform der Erfindung wird eine Mutation auf einem oder mehreren der cis-aktiven Abschnitte des viralen DNA-Genoms eingeführt, wobei die Mutation die Verpackung des viralen DNA-Genoms verändert.

In einer erfindungsgemäß besonders bevorzugten Ausgestaltung der Erfindung werden die prokaryonten Anteile des DNA-Abschnitts im Schritts (b) so ausgewählt, daß sie zumindest teilweise zueinander homologe Sequenzen aufweisen, die eine solche Länge besitzen, daß eine homologe Rekombination ermöglicht wird, wodurch die prokaryonten Anteile des DNA-Abschnitts aus (b) eliminiert werden. Durch eine derartige homologe Rekombination werden die im erfindungsgemäßen Verfahren eingeführten prokaryonten Anteile in nachfolgenden Verfahrensschritten wieder eliminiert. Hierdurch können beispielsweise Antibiotikaresistenzen, die durch das Einbringen des DNA-Abschnitts in die eukaryontische Zielzelle eingeführt wurden und weitere prokaryonte Anteile wieder entfernt werden, so daß ein für die Gentherapie verwendbarer DNA-Virus-Vektor zur Verfügung steht, der einen möglichst geringen Anteil prokaryonter Fremdsequenzen besitzt. Ein Beispiel hierfür wird in den Ausführungsbeispielen näher beschrieben.

Weiterhin wird erfindungsgemäß ein DNA-Virus-Vektor bereitgestellt, der, in funktioneller Anordnung, zumindest ein DNA-Virus-Genom ≥ 100 kbp. zumindest ein in prokaryontischen Zellen selektierbares Markergen und die Information für die Replikation des viralen DNA-Genoms in prokaryontischen Zellen umfaßt.

Nachfolgend wird die Erfindung anhand der Abbildungen und der Ausführungsbeispiele weiter beschrieben. Die Ausführungsbeispiele stellen bevorzugte Ausgestaltungen der Erfindung dar. Die Erfindung ist jedoch nicht auf diese speziellen Beispiele beschränkt.

Die beiliegenden Abbildungen zeigen:
- **Abb. 1:**: Klonierung genomischer 895.8 DNA in E. coli mit Hilfe eines F-Faktor Plasmids.
- **Abb. 2:**: Klonierung genomischer P3HR1 DNA ohne Verpackungssignale (TR) in E. coli mit Hilfe eines F-Faktor-Plasmids.
- **Abb. 3:**: Klonierung genomischer P3HR1 DNA in E. coli mit Hilfe eines F-Faktor-Plasmids.
- **Abb. 4:**: Einführen von Mutationen in F-Faktor klonierte genomische EBV DNA durch Allelaustausch
- **Abb. 5:**: Einführen von Mutationen in F-Faktor klonierte genomische EBV DNA durch selektive Integration

Es wurden in zwei verschiedene Zellinien, die latent mit zwei verschiedenen EBV Stämmen infiziert sind (B95.8 und P3HR1/HH514), ein Abschnitt des E. coli Plasmids F-Faktor eingeführt, der die Replikationsfunktionen und einen selektionierbaren Marker für E. coli umfaßt. Dieser lineare Abschnitt des F-Faktor Plasmids wurde jeweils links und rechts mit EBV Abschnitten flankiert, so daß der F-Faktorabschnitt über zwei homologe Rekombinationsereignisse in das endogene EBV Genom in den Zellinien targetiert werden kann. Zusätzlich zu den flankierenden EBV Abschnitten enthält das DNA Fragment mindestens ein Markergen, das zur Selektion (z B. Hygromycinphosphotransferase) oder phänotypischen Charakterisierung (z. B. Green Fluorescence Protein. GFP) in eukaryonten Zellen geeignet ist. Dieses Konstrukt wird in EBV-infizierte Zellen transfiziert. es kommt zur homologen Rekombination mit dem endogenen EBV Genom und der zielgerichteten Integration des F-Faktor Abschnitts zusammen mit dem Markergen. Die so veränderte Zellinien tragen nun ein rekombinantes EBV Genom, das in einem Shuttlesystem in E. coli transferiert werden kann. Das rekombinante EBV Genom repliziert in E. coli über den F-Faktor Anteil, der auch den prokaryonten Selektionsmarker trägt. Weitere genetische Veränderungen des rekombinanten EBV Genoms können nun mit herkömmlichen genetischen Techniken in E. coli stattfinden.

Das rekombinante EBV Genom kann aus E. coli isoliert werden und die DNA Moleküle können über herkömmliche Techniken präpariert werden. Diese DNA Moleküle werden nun über DNA Transfertechniken in eukaryonte Zellen stabil oder transient eingeführt. Die rekombinanten EBV Genome replizieren extrachromosomal in diesen Zellen wie in den latent infizierten Zellinien B95.8 und P3HR1/HH514. Spontan oder nach chemischer oder anderweitiger Induktion der lytischen Phase von EBV kommt es zur Synthese von rekombinanten Epstein-Barr Virionen, die in den Zellkulturüberstand freigesetzt werden und die für weitere Anwendungen zur Verfügung stehen.

### Verfahren zur Herstellung

In das Genom des B95.8 Virus wurde mit dem oben beschriebenen Verfahren ein F-Faktor-Abschnitt eingeführt, der den eukaryonten Selektionsmarker Hygromycinphosphotransferase und das phänotypische Markergen GFP neben den funktionellen F-Faktor-Komponenten enthält. Das rekombinante 895.8 Genom wurde erzeugt, in E. coli transferiert und rekombinante EBV DNA aus E. coli präpariert. Diese DNA wurde in verschiedene EBV-negative Zellen (293. EBV-negative Akata, neuronale Zellen) eingeführt. Die Induktion des lytischen Zyklus führt zur Freisetzung von infektiösen Virionen, die als genetische Information rekombinantes EBV Genom enthalten. Diese rekombinanten EBV Genome sind z. B. in der Lage, primäre humane B-Lymphozyten zu immortalisieren.

In das Genom des P3HR1/HH514 Virus wurden in zwei unabhängigen Experimenten zwei verschiedene F-Faktor-Abschnitte in zwei unterschiedliche Genomorte eingeführt. Die Targetierung für diese Genomorte erfolgte durch unterschiedliche flankierende EBV-Abschnitte. Es wurde in einem Experiment ein weiteres EBV Gen eingeführt, in dem zweiten Experiment wurde ein EBV Abschnitt durch die Wahl der flankierenden EBV-Abschnitte gezielt deletiert.

Die rekombinanten Genome des B95.8 als auch des P3HR1/HH514 Virus wurde weiter in E. coli durch Deletionen oder Einfügen neuer Gene modifiziert.

### Ausführungsbeispiele

### 1. Klonierung eines voli funktionsfähigen Genoms des B95.8 Stammes

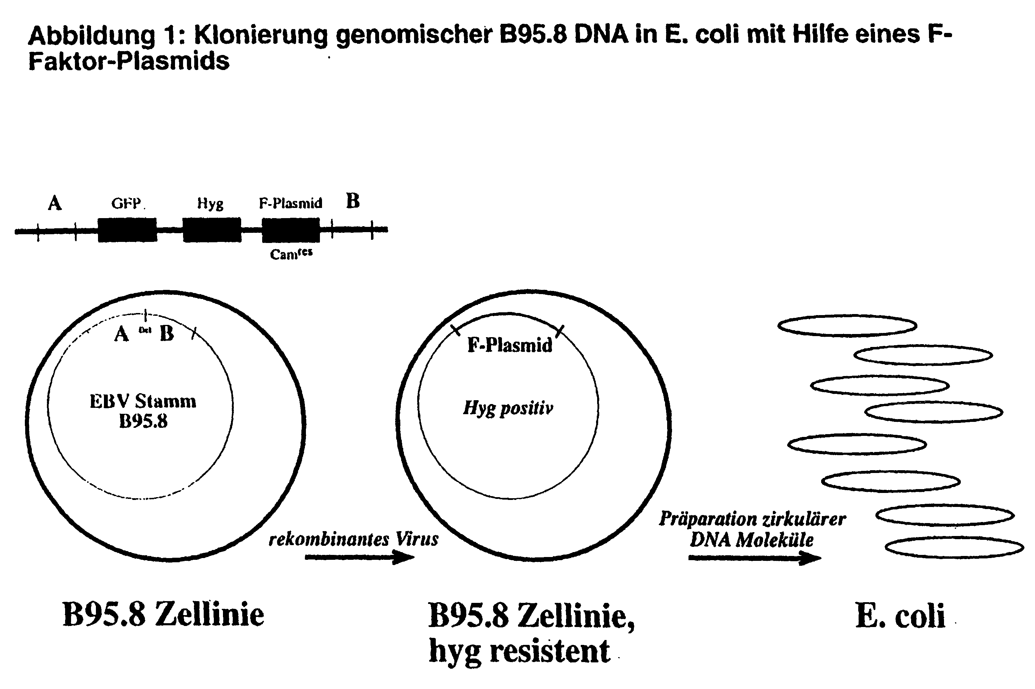

Ein F-Faktor Plasmid. mit p1944.12 bezeichnet (Tabelle 1).wurde in E. coli über konventionelle DNA-Klonierungstechniken hergestellt. Wie in Abbildung 1 schematisch gezeigt, enthält das Plasmid zwei Abschnitte A und B, die subgenomische Abschnitte des B95.8 Genoms darstellen. Die Nukleotidsequenzkoordinaten dieser Abschnitte sind in Tabelle 1 angegeben. Die Abschnitte A und B flankieren das pMBO131 Replicon (O'Connor *et al*., 1989), das den prokaryonten Replikationsursprung des F-Faktors zusammen mit dem selektionierbaren prokaryonten Markergen Chloramphenicolacetyltransferase (cam) umfaßt. Weiterhin ist das eukaryonte Markergen Hygromycinphosphotransferase (Hyg), das vom SV40 early Promotor/Enhancer exprimiert wird, und das Markergen Green Fluorescence Protein (GFP), das vom immediate early Promotor/Enhancer des humanen Cytomegalovirus ist, in dem Plasmid p1944.12 enthalten (Tabelle 1 und Abbildung 1). Funktionswichtige Teile des Plasmids p1944.12 sind die EBV-Abschnitte, der pMBO131 Anteil und das Gen Hyg, GFP sind optional.

Die Abschnitte A und B des B95.8 Genoms auf p1944.12 sind so gewählt, daß sie die natürliche Deletion im Genom von B95.8 links und rechts flankieren. Um dieses Plasmid in das Genom von B95.8 einzuführen, wurde es mit dem Restriktionsenzym NotI linearisiert und die freien DNA-Enden mit sogenannten Hairpin-Oligonukleotiden mit Hilfe von T4-DNA-Ligase versiegelt, um sie gegen Exonukleasen zu schützen. Die so veränderte Plasmid DNA wurde über Elektroporation in die B95.8 Zellen transfiziert und die Zellen in Gegenwart von 100 µg/ml Hygromycin selektioniert. Unter diesen Selektionsbedingungen überleben nur solche Zellen, die die DNA von p1944. 12 aufgenommen und entweder in das Wirtszellgenom oder in die extrachromosomalen Genomkopien des B95.8 EBV Stammes integriert haben. Um zwischen diesen beiden Möglichkeiten zu unterscheiden, wurden Einzelzellklone mittels Gardella-Agarose-Gelen und anschließender Southernblothybridisierung untersucht. Es gelang, über diese Analysetechniken eine Reihe von Zellklonen zu identifizieren, die p1944.12 in die Genomkopien des B95.8 EBV Stammes integriert hatten.

Um diese genetisch veränderten EBV Genome in E.coli zu klonieren, wurde Plasmid DNA aus den Zellklonen isoliert, mit Hilfe von Elektroporationsmethoden in den E.coli Stamm DH10B transferiert und über die Resistenz gegen Chloramphenicol selectioniert. Die anschließende Isolierung von Plasmid DNA aus E.coli ergab DNA Präparationen, die nach Spaltung mit verschiedenen Restriktionsenzymen DNA Fragmente zeigten, die exakt der Komposition von B 98.8 DNA einschließlich integriertem p1944.12 Anteil entsprachen. Die Gesamtgröße dieser EBV Genome liegt über 170 kbp und wurde mit 2010 bezeichnet.

2010 DNA wurde im Mikrogrammaßstab aus E.coli isoliert und über verschiedene DNA Transfektionsmethoden in EBV-negative eukaryonte Zellen eingebracht. Diese Zellen können beispielsweise EBV-negative Akatazellen, 293- und Fibroblastenzellen oder -zellinien sein. Diese Zellen wurden unter angepaßten Hygromycinkonzentrationen selektioniert, um sicherzustellen, daß 2010 DNA in die Zellen stabil eingebracht worden war. Die lytische Phase von EBV wurde über verschiedene Techniken induziert, z. B. durch Transfektion des BZLF1 Expressionsplasmids pCMV-BZLF1 (Hammerschmidt and Sugden. 1988). Die Induktion der lytischen Phase von EBV in diesen Zellen führt zur Freisetzung von infektiösen Viren, die zur Immortalisation von primären menschlichen B-Lymphozyten in der Lage sind. Um diese Eigenschaft von 2010 EBV nachzuweisen, wurde zellfreier Überstand aus lytisch induzierten Zellen gewonnen und zur Infektion von primären menschlichen B-Lymphozyten eingesetzt. Vier bis sechs Wochen nach Infektion dieser Zellen konnten permant proliferierende Zellinien etabliert werden, die 2010 EBV DNA enthielten, wie über verschiedene Techniken (Southern-Blot-Hybridisierung, PCR-Analyse) nachgewiesen wurde.

### 2. Klonierung eines verpackungsdefekten P3HR1/HH514 Genoms in E. coli

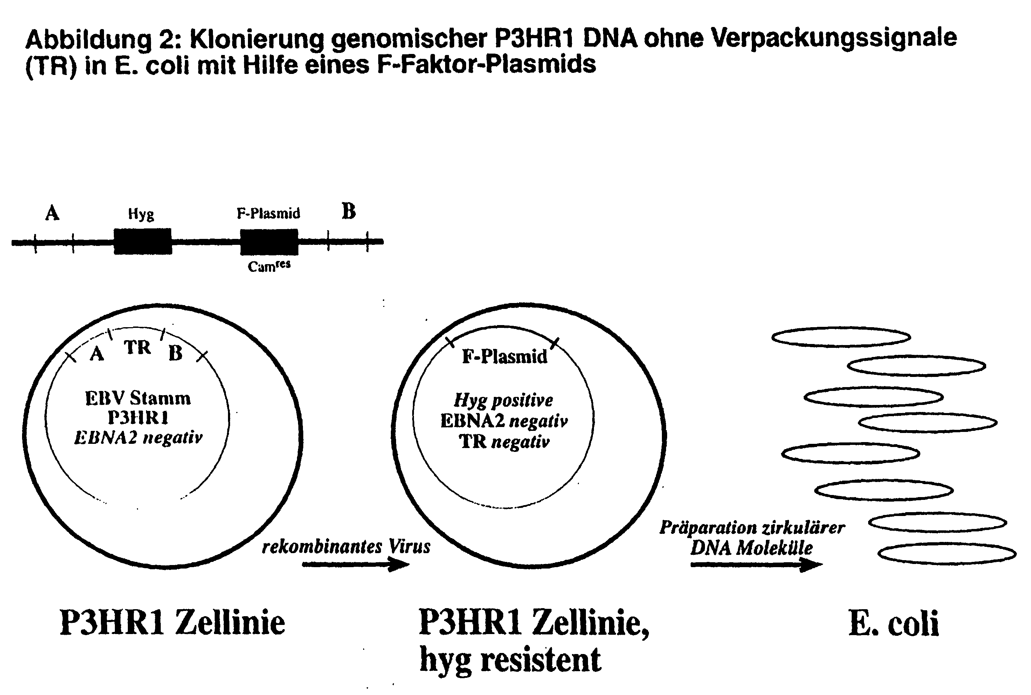

Ein F-Faktor Plasmid, mit p2061.2 bezeichnet (Tabelle 1), wurde in E. coli über konventionelle DNA-Klonierungstechniken hergestellt. Wie in Abbildung 2 schematisch gezeigt, enthält das Plasmid zwei Abschnitte A und B, die subgenomische Abschnitte des P3HR1/HH514 Genoms darstellen. Die Nukleotidsequenzkoordinaten dieser Abschnitte sind in Tabelle 1 angegeben. Die Abschnitte A und B flankieren das pMBO131 Replicon .(O'Connor *et al*., 1989)., das den prokaryonten Replikationsursprung des F-Faktors zusammen mit dem selektionierbaren prokaryonten Markergen Chloramphenicolacetyltransferase (cam) umfaßt. Weiterhin ist das eukaryonte Markergen Hygromycinphosphotransferase (Hyg), das vom SV40 early Promotor/Enhancer exprimiert wird, in dem Plasmid p2061.2 enthalten (Tabelle 1 und Abbildung 2). Funktionswichtige Teile des Plasmids p2061.2 sind die EBV-Abschnitte. der pMBO131/F-Faktor-Anteil und das Gen Hyg.

Die Abschnitte A und B des P3HR1/HH514 Genoms auf p2061.2 sind so gewählt, daß sie die 'terminal repeats' im Genom von P3HR1/HH514 links und rechts flankieren. Die 'terminal repeats' (TR) sind u.a. dadurch charakterisiert, daß sie die Signalsequenzen tragen, die für die Verpackung von viraler genomischer DNA in EBV Kapside notwendig sind. Die Konstruktion von p2061.2 hat zum Ziel, die TR Signalsequenzen zu deletieren und durch die Anteile pMBO131 und Hyg von p2061.2 zu ersetzen. Um p2061.2 in das Genom von P3HR1/HH514 einzuführen wurde es mit dem Restriktionsenzym Sacl linearisiert und die freien DNA-Enden mit sogenannten Hairpin-Oligonukleotiden mit Hilfe von T4-DNA-Ligase versiegelt. um sie gegen Exonukleasen zu schützen. Die so veränderte Plasmid DNA wurde über Elektroporation in die P3HR1/HH514 Zellen transfiziert und die Zellen in Gegenwart von 200 µg/ml Hygromycin selektioniert. Unter diesen Selektionsbedingungen überleben nur solche Zellen, die die DNA von p2061.2 aufgenommen und entweder in das Wirtszellgenom oder in die extrachromosomalen Genomkopien des P3HR1/HH514 EBV Stammes integriert haben. Um zwischen diesen beiden Möglichkeiten zu unterscheiden, wurden Einzelzellklone mittels Gardella-Agarose- Gele und anschließender Southernblothybridisierung untersucht. Es gelang, über diese Analysetechniken eine Reihe von Zellklonen zu identifizieren, die p2061.2 in die Genomkopien des P3HR1/HH514 EBV Stammes integriert hatten.

Um diese genetisch veränderten EBV Genome in E. coli zu klonieren, wurde Plasmid DNA aus den Zellklonen isoliert. mit Hilfe von Elektroporationsmethoden in den E. coli Stamm DH10B transferiert und über die Resistenz gegen Chloramphenicol selektioniert. Die anschließende Isolierung von Plasmid DNA aus E. coli ergab DNA Präparationen, die nach Spaltung mit verschiedenen Restriktionsenzymen DNA Fragmente zeigten, die exakt der Komposition von P3HR1/HH514 genomischer DNA ohne den TR Signalsequenzen, aber einschließlich integriertem p2061.2 Anteil, entsprachen. Die Gesamtgröße dieser EBV Genome liegt über 170 kbp und wurde mit 2087.2a bezeichnet.

2087.2a DNA wurde im Mikrogrammaßstab isoliert und über verschiedene DNA Transfektionsmethoden in EBV-negative eukaryonte Zellen eingebracht. Diese Zellen können beispielsweise EBV-negative Akatazellen, 293- und Fibroblastenzellen oder -zellinien sein. Diese Zellen wurden unter angepaßten Hygromycinkonzentrationen selektioniert, um sicherzustellen, daß 2087.2a DNA in die Zellen stabil eingebracht worden war. Die lytische Phase von EBV wurde über verschiedene Techniken induziert, z. B. durch Transfektion des BZLF1 Expressionsplasmids pCMV-BZLF1 .(Hammerschmidt and Sugden, 1988).. Im Gegensatz zu dem unter 1. beschriebenen Ausführungsbeispiel führt die Induktion der lytischen Phase von EBV in diesen Zellen nicht zur Freisetzung von infektiösen Viren, da die Verpackung von EBV genomischer DNA durch das Fehlen der TR-Verpackungssignale unterbleibt. Diese Zellinien stellen aber alle viralen Funktionen zur Verpackung von DNA Molekülen in trans zur Verfügung, die z. B. für die Enkapsidierung von p554 .(Hammerschmidt and Sugden, 1989). oder mini-EBV-Plasmiden .(Kempkes *et al..* 1995a: Kempkes *et al*., 1995b). notwendig sind, ohne daß sogenanntes Helfervirus freigesetzt wird (Hammerschmidt and Sugden. 1989)..

### 3. Klonierung eines P3HR1/HH514 Genoms mit genetischer Komplementation in E. coli.

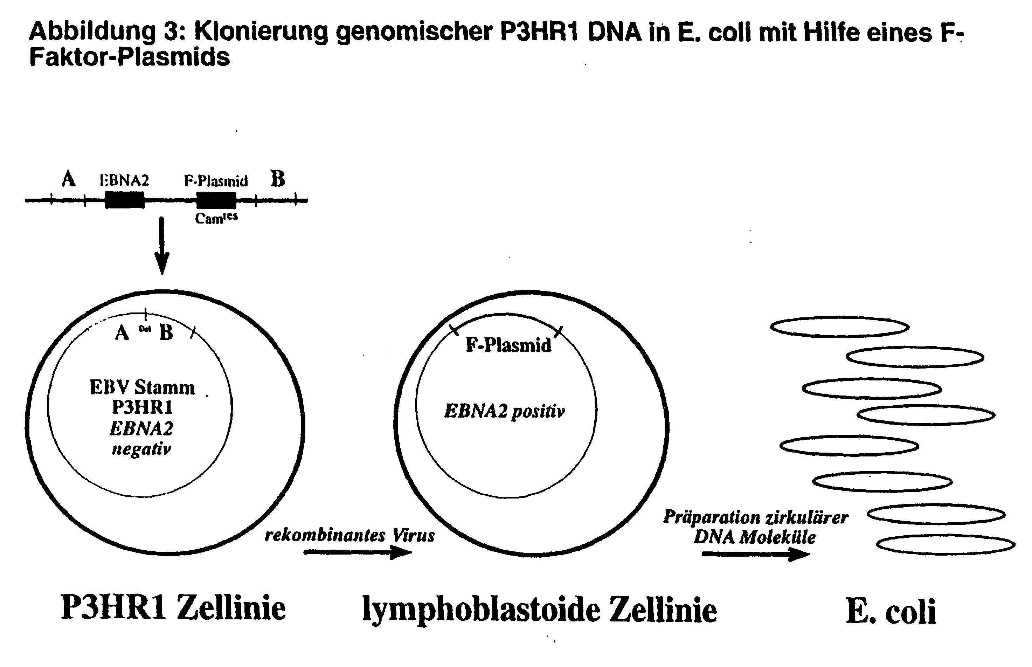

Ein F-Faktor Plasmid, mit p1820.15 bezeichnet (Tabelle 1) wurde in E. coli über konventionelle DNA-Klonierungstechniken hergestellt. Wie in Abbildung 3 schematisch gezeigt, enthält das Plasmid zwei Abschnitte A und B. die subgenomische Abschnitte des B95.8 Genoms darstellen. Die Nukleotidsequenzkoordinaten dieser Abschnitte sind in Tabelle 1 angegeben. Die Abschnitte A und B flankieren das pMBO131 Replicon .(O'Connor *et al*., 1989)., das den prokaryonten Replikationsursprung des F-Faktors zusammen mit dem selektionierbaren prokaryonten Markergen Chloramphenicolacetyltransferase (cam) umfaßt. Weiterhin sind als funktionelle Abschnitte in dem Plasmid p1820.15 das EBV Gen EBNA2 und zwei Exons des EBV Gens EBNA-LP enthalten .(Hammerschmidt und Sugden, 1989).(Tabelle 1 und Abbildung 3). Funktionswichtige Teile des Plasmids p1820.15 sind die flankierenden EBV-Abschnitte, das Gen EBNA2 und Anteile des Gens EBNA-LP und der pMBO131 Anteil.

Die Abschnitte A und B des B95.8 Genoms auf p1820.15 sind so gewählt, daß sie die Deletion im Genom von P3HR1/HH514 links und rechts flankieren, die zwei Exons von EBNA-LP und das gesamte EBNA2 Gen umfaßt. Um dieses Plasmid in das Genom von P3HR1/HH514 einzuführen, wurde es mit dem Restriktionsenzym Nhel linearisiert und die freien DNA-Enden mit sogenannten Hairpin-Oligonukleotiden mit Hilfe von T4-DNA-Ligase versiegelt, um sie gegen Exonukleasen zu schützen. Die so veränderte Plasmid DNA wurde über Elektroporation in die P3HR1/HH514 Zellen transfiziert zusammen mit dem Expressionsplasmid pCMV-BZLF1, das den lytischen Zyklus von EBV in diesen Zellen induziert. Zellfreier Kulturüberstand wurde gewonnen und zur Infektion von primären menschlichen B-Lymphozyten eingesetzt. Vier bis sechs Wochen nach Infektion dieser Zellen konnten permanent proliferiende Zellinien etabliert werden. Die Deletion in P3HR1/HH514, die das gesamte EBNA2 Gen und zwei Exons des EBNA-LP Gens umfaßt, unterbindet die B-Zell-Immortalisation. Die Abschnitte auf dem Plasmid p1820.15 komplementieren die Deletion im Genom von P3HR1/HH514 Virus über die homologen Rekombination zwischen P3HR1/HH514 DNA und p1820.15 DNA, ähnlich wie beschrieben (Hammerschmidt and Sugden, 1989). Die homolog rekombinierten EBV Genome zeichnen sich durch die wiedererlangte Fähigkeit aus. primäre menschliche B-Lymphozyten zu immortalisieren. Gleichzeitig kommt es zur Integration des F-Faktoranteils in p1820.15. so daß das Replicon pMBO131 Bestandteil des rekombinanten EBV Genoms wird.

Um diese genetisch veränderten EBV Genome in E. coli zu klonieren, wurde Plasmid DNA aus den Zellklonen isoliert. mit Hilfe von Elektroporationsmethoden in den E. coli Stamm DH10B transferiert und über die Resistenz gegen Chloramphenicol selektioniert. Die anschließende Isolierung von Plasmid DNA aus E. coli ergab DNA Präparationen. die nach Spaltung mit verschiedenen Restriktionsenzymen DNA Fragmente zeigten, die exakt der Komposition von P3HR1/HH514 genomischer DNA einschließlich dem integriertem Anteil von p1820.15 entsprachen. Die Gesamtgröße dieser EBV Genome liegt über 170 kbp und wurde mit 1947 (K-Klon) bezeichnet.

### 4. Einführen von Mutationen in F-Faktor klonierte genomische EBV DNA durch Allelaustausch

Die Klonierung von genomischer EBV DNA in E. coli erlaubt die einfache genetische Modifikation durch etablierte bzw. an die erfindungsgemäßen, speziellen Bedingungen angepaßte Methoden.

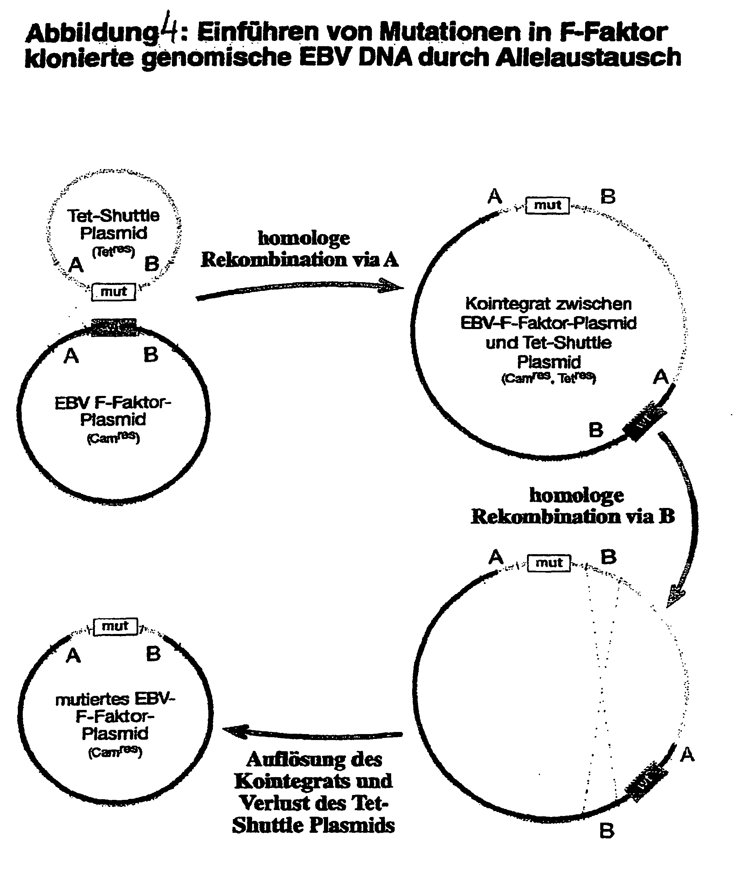

Zum Austausch der Verpackungssignale in dem klonierten EBV Genom 1947 (Tabelle 1) wurde das rekombinante Plasmid p2060.1 in E. coli hergestellt, das aus folgenden Komponenten besteht (siehe Abb. 4):
1. Einem prokaryonten Vektorplasmid mit temperatursensitivem Replikationsursprung (Tet-Shuttle Plasmid) und dem prokaryonten Selektionsmarker Tetracyclinresistenz. z. B. auf der Basis von pMBO96 .(O'Connor *et al*., 1989)..
2. Flankierenden Abschnitten A und B, die die EBV Sequenzen des P3HR1/HH514 Stammes von Nukleotidposition #165.840 bis #169,924 (A) und #1 bis #3955 (B) enthalten.
3. Dem eukaryonten Selektionsmarker Hygromycinphosphotransferase, exprimiert vom SV40 early Promoter/Enhancer. Dieses Gen liegt zwischen den EBV Abschnitten A und B und ist in Abb. 4 mit "mut" bezeichnet.

Das F-Faktor-Plasmid 1947 (Tabelle 1) wird in einen rekombinationskompetenten (recA+) E. coli Stamm transfektiert, und die Transfektanten werden durch ihre Chloramphenicolresistenz identifiziert. In einem zweiten Schritt wird das Tet-Shuttle-Plasmid p2060.1 in die 1947 tragenden Transfektanten transfektiert und die Bakterien bei 30°C auf die Doppelresistenz gegen Chloramphenicol und Tetracyclin selektiert. Die beiden Plasmide replizieren unabhängig voneinander in derselben E. coli Zelle. Die homologe Rekombination via A (kann auch via 8 erfolgen) wird durch Erhöhung der Temperatur auf 42°C und Selektion auf Resistenz gegen Chloramphenicol und Tetracyclin erzwungen, da das Tet-Shuttle-Plasmid p2060.1 bei dieser Temperatur nicht mehr replizieren kann. Ein Kointegrat entsteht wie in Abb. 4 gezeigt. Mit einer weiteren homologen Rekombination. diesmal via B. erfolgt die Auflösung des Kointegrats und der Verlust des Tet-Shuttle-Plasmids, so daß die Veränderung "mut" (in diesem Fall das Gen Hygromycinphosphotransferase) die EBV Sequenz "wt" (in diesem Fall die Verpackungssignale TR) ersetzt. Der letzte Schritt erfolgt nach statistischer Verteilung, d. h. bei der Auflösung des Kointegrats kann entweder die Mutation "mut" eingeführt werden (wie in Abb. 4 gezeigt) oder die Ausgangssitutation "wt" wieder entstehen.

Vorteil dieser Methode gegenüber der unter 5. beschriebenen ist die Möglichkeit, ohne Einführen von weiteren Fremdsequenzen ganz gezielt auch einzelne Nukleotide im klonierten EBV-Genom auszutauschen.

### 5. Einführen von Mutationen in F-Faktor klonierte genomische EBV DNA durch selektive Integration

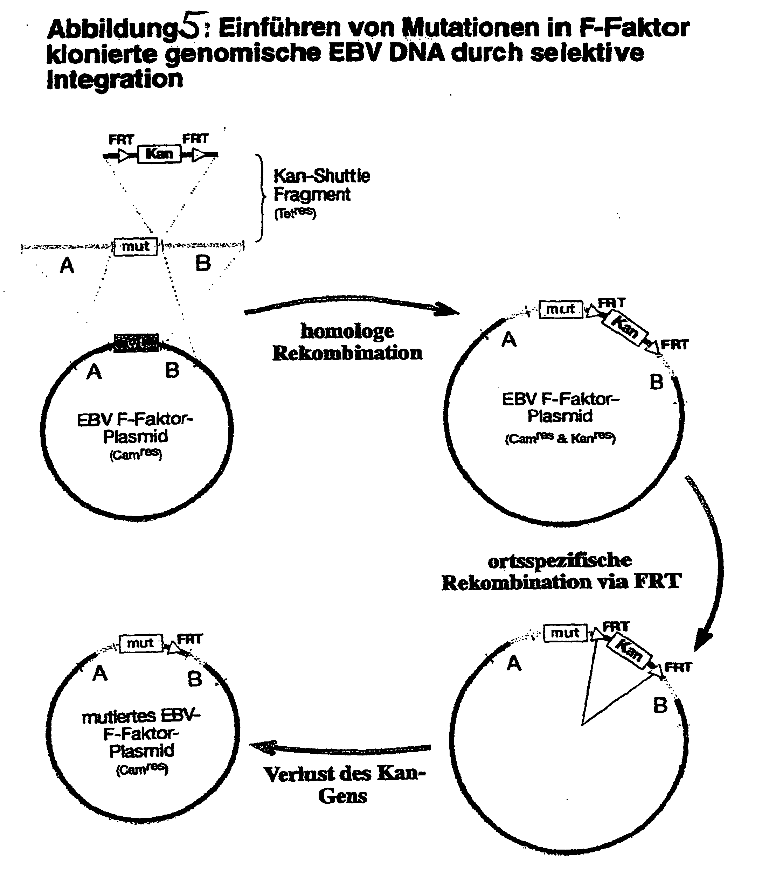

Eine zweite, alternative Methode zur genetischen Modifikation von F-Faktor klonierter genomischer EBV DNA besteht in der Integration von linearen DNA Fragmenten durch homologe Rekombination, wie in Abbildung 5 schematisch gezeigt.

Zur Mutation des EBV Gens LMP2A wird zuerst in einem gewöhnlichen, von pBR abgeleiteten Plasmid ein. Kan-Shuttle-Fragment kloniert, wie in Abb. 5 gezeigt. Das Kan-Shuttle-Fragment in dem Plasmid p2120 enthält zwei EBV Abschnitte A und B, die die EBV Sequenzen des B95.8 Stammes von Nukleotidposition #163,473 bis #166,180 (A) und #166,938 bis #172.281/#1 bis #649 (B) enthalten. Die in Abb. 5 bezeichnete Mutation "mut" besteht aus zwei sogenannten IoxP Sequenzmotiven, die ein Exon des EBV Gens LMP2A (EBV Nukleotidpositionen #166,181 bis #166,937) flankieren. Anschließend an eine der beiden IoxP Sequenzmotive ist ein mit FRT Sequenzmotiven begrenzter Selektionsmarker für die Resistenz gegen das Antibiotikum Kanamycin kloniert. Das Fragment, wie in Abb. 5 oben links dargestellt, wird durch Restriktionsenzyme vom pBR Anteil getrennt und die lineare Kan-Shuttle-Fragment-DNA isoliert. Die Kotransfektion dieses linearen DNA-Fragments zusammen mit dem F-Faktor klonierten EBV Genom 2010 erfolgt in einen E. coli Stamm, der vorzugsweise Exonuklease V negativ ist; z. B. den E. coli Stamm BJ5183 .(Hanahan, 1983). Die Selektion der transfektierten Bakterien auf Resistenz gegen Chloramphenicol und Kanamycin erzwingt die homologe Rekombination zwischen beiden DNA Molekülen und ergibt idealerweise das oben rechts gezeigte EBV F-Faktor-Plasmid. Das Kanamycinresistenzgen kann durch ortspezifische Rekombination via FRT über die Rekombinase FLP in E. coli entfemt werden .(Cherepanov and Wackernagel, 1995). Als Ergebnis entsteht ein mutiertes EBV-F-Faktor-Plasmid. Der prinzipielle Unterschied zu der Situation unter 4, ist der Verbleib von Fremdsequenzen, nämlicher eines FRT Sequenzmotivs.

Antibiotikaresistenzmarker und Sequenzen, die für die ortspezifische Rekombination eingesetzt werden, sind beliebig wählbar, was gleichermaßen auch für den Ansatz unter 4, gilt.

Des weiteren ist die Erstellung einer Genombank denkbar, die durch Transposon-vermittelte Mutagenese in E. coli hergestellt werden kann und die damit eine beliebig große Anzahl an individuell mutierten EBV Genomen repräsentiert. Durch die zufällige Insertion der Transposons in die klonierten EBV Genome kommt es an der Insertionsstelle zur Mutagenese, z. B. zur Unterbrechung des Leserahmens eines EBV Gens. Der Vorteil einer solchen EBV-Genombank wäre die Möglichkeit, auf bestimmte Phänotypen biologisch selektionieren zu können. Eine Genombank mit mutierten herpesviralen Genomen könnte z. B. als Screeningsystem für antivirale Substanzen eingesetzt werden.

Das erfindungsgemäße Verfahren kann somit noch wie folgt abgewandelt werden:
(f) Einführen des rekombinanten DNA-Virus-Vektor-Genoms in eine Prokaryontenzelle;
(g) Einführen eines Plasmids in die Prokaryontenzelle, das, flankiert von DNA-Abschnitten in einer solchen Länge, die eine Rekombination mit dem DNA-Virus-Vektor-Genom ermöglichen, zumindest, in funktioneller Anordnung, eine DNA-Sequenz, die im Vergleich zu der auf dem DNA-Virus-Vektor-Genom vorliegenden homologen Sequenz eine Mutation enthält, und ein selektierbares Markergen enthält.
(h) Integration der unter (g) beschriebenen DNA-Sequenz und das Markergens durch Rekombination in das DNA-Virus-Genom;
(i) wahlweise Aufreinigen und Isolieren des rekombinanten DNA-Vektors.

In einer weiteren Ausführungsform enthält das Plasmid im Schritt (g) weiterhin von einer Rekombinase erkennbare Sequenzmotive, und vor dem Schritt (e) wird das durch die Rekombination eingeführte Resistenzgen durch ortsspezifische Rekombination über die von der Rekombinase erkannten Sequenzmotive wieder entfernt.

Entfernung des prokaryonten Replikons und anderer Fremdanteile aus herpesviralen Genomen kloniert in E.coli.

Bei der Anwendung von Genvektoren ist es wünschenswert, den Anteil der genetischen Information, der in die Zielzelle eingebracht wird, so klein wie möglich zu halten und auf das wesentliche zu beschränken. Insbesondere sind subgenomische Abschnitte unerwünscht, die prokaryonte Markergene wie Antibiotikaresistenzen tragen oder aber ein potentielles Sicherheitsrisiko darstellen, da sie Anlaß für homolge Rekombinationen mit verschiedenen Bakterien sein könnten. Die mit Hilfe eines F-Faktor Replikons klonierten EBV-Genome stellen ein solch hybrides Molekül dar. Der F-Faktoranteil ist bei der Propagierung des DNA-Moleküls in E. coli essentiell, er ist aber völlig funktionslos in der eukaryonten Zelle, in die das in E. coli modifizierte EBV/F-Faktor Replikon eingeführt wird, um genetisch modifiziertes EBV herzustellen.

Es wurde eine überraschend einfache Methode gefunden, um diesen F-Faktoranteil gänzlich und ohne weitere genetische Manipulation zu entfernen.

Bei der Klonierung eines voll funktionsfähigen Genoms des B95.8 Stammes in E. coli wurde ein F-Faktor Plasmid verwendet, das mit p1944.12 bezeichnet wurde (Tabelle 1). Es wurde in E. coli über konventionelle DNA-Klonierungstechniken hergestellt. Wie in Abbildung 1 schematisch gezeigt, besteht das Plasmid aus zwei Abschnitten A und B, die subgenomische Abschnitte des B95.8 -Genoms darstellen. Die Nukleotidsequenzkoordinaten dieser Abschnitte sind in Tabelle 1 angegeben; sie erstrecken sich von EBV-Koordinate #143.458 bis #152.636 im Abschnitt A und von #149.930 bis #159.880 im Abschnitt B. Die Abschnitte A und B flankieren das pMBO131-Replicon (O'Connor et al., 1989), das den prokaryonten Replikationsursprung des F-Faktors zusammen mit dem selektionierbaren, prokaryonten Markergen Chloramphenicolacetyltransferase (cam) und anderen Genen umfaßt. Die beiden EBV-Anteile sind so gewählt, daß sie teilweise die gleichen DNA-Sequenzabschnitte tragen, die im konkreten Beispiel eine partielle Duplikation von 2.7 kbp darstellen (von #149.930 bis #152.636). Das aus diesen Experimenten in E. coli klonierte F-Faktor-Plasmid wurde mit 2010 bezeichnet (Tabelle 1). Nach Transfektion der 2010 DNA in 293 Zellen kommt es spontan zu homolgen Rekombinationsereignissen zwischen den duplizierten Anteilen in den Abschnitten A und B in diesen Zellen und zur Deletion aller Sequenzen, die aus den prokaryonten Klonierungsschritten stammen.

Als weitere denkbare Anwendungen hier einige Beispiele:
- Etablierung von gezielt veränderten DNA-Viren, denen bestimmte Virulenzgene fehlen und die als gezielt attenuierte Vakzinestämme eingesetzt werden können. Als Virulenzgene kommen im Fall von EBV z.B. EBNA2, LMP1, LMP2A, u. a. in Frage, die für die Attenuierung eines solchen Vakzinestammes deletiert werden könnten.
- Generierung von rekombinanten EBV Vektoren, die in Verpackungszellinien mit einer EBV-Hülle versehen werden und die zum Gentransfer in Zellen des Menschen oder anderer Mammalier in vitro oder in vivo eingesetzt werden können. Diese Verpackungszellinien enthalten stabil ein EBV Genom oder dessen wichtige Teile, die für die Produktion viraler Strukturproteine nötig sind, um EBV Vektoren zu verpacken. Die Verpackungszellinien werden vorzugsweise mit einem EBV Genom versehen, dem die eigenen Verpackungssignale fehlen (siehe Abb. 2 und Ausführungen), um das Freisetzen von unerwünschten, sogenannten Helferviren zu verhindern. Die verpackbaren EBV Vektoren können auf herkömmlichen rekombinanten DNA Plasmiden beruhen, die alle Elemente enthalten, die für die Amplifikation ihrer DNA (oriLyt), deren Verpackung (TR), und der stabilen extrachromosomalen Existenz in der Rezipientenzelle (oriP und EBNA1) nötig sind. Zusätzlich können diese Vektoren therapeutisch interessante Gene, z.B. Faktor VIII, im Sinne einer Genkorrektur tragen.

Darüber hinaus kann die Affinität dieser Vektoren für bestimmte Zielzellen verändert werden. EBV Glykoproteine, die für die Infektion bestimmter Zellen (natürlicherweise B-Zellen und einige Epithelzellen) verantwortlich sind, können durch Mutation der F-Faktor klonierten EBV Genome in E. coli verändert oder gegen andere ausgetauscht werden. Ein Beispiel für einen erweiterten Zelltropismus wäre das Vesikulostomatitisvirus-Glykoprotein G, ein Beispiel für den extrem spezifischen Zelltropismus für fast ausschließlich pluripotente hämatopoetische Stammzellen wäre der Ligand für den Stem Cell Factor Receptor auf der Stammzelle, der membranständige Stem Cell Factor Ligand (mSCF-ligand). Dieses sogenannte Pseudotyping ist besonders für herpesvirale Vektoren attraktiv, da die für die Infektiosität wichtigen Glykoproteine in einer von der Kernmembran abgeleiteten Phospholipidmembran eingebettet sind Diese Membran ist nicht wesentlich strukturiert und umhüllt das streng geometrische Kapsid des Virions nur locker. Im Gegensatz zu vielen kleineren Viren (z.B. Adenovirus, Adenoassoziiertes Virus (AAV), Retroviren) ist die Hülle von Herpesviren und anderen großen DNA Viren flexibel und verlangt keine bestimmten Proteinkonfigurationen oder - faltungen, die anderenfalls die Virusreifung und -assemblierung verhindern.

### Vorteile gegenüber dem Stand der Technik

Das beschriebene Verfahren erlaubt beispielsweise die Herstellung von rekombinanten Herpesvirusgenomen, die z.B. in E. coli klonal vermehrt werden können. Mit Sicherheit kann man davon ausgehen, daß dieses Verfahren auf alle großen DNA Viren angewandt werden kann (z. B. Poxviren, Iridioviren, andere Herpesviren). Die Klonierung dieser Virusgenome z.B. in E. coli erlaubt die einfache Modifikation durch gezielte Veränderungen viraler Gene, ihre Deletion oder Hinzufügen von weiteren Genen, die von Interesse sind. Die so hergestellten viralen Genome weisen je nach genetischer Modifikation neue Eigenschaften auf, die z. B. die Expression von Proteinen in den jeweiligen Zielzellen der Viren erlauben.

Die in E. coli klonierten viralen Genome stellen ein Produkt dar, das z. B. für die Enkapsidierung von rekombinanten Plasmiden oder subgenomischen viralen Abschnitten geeignet ist.

### LITERATURLISTE

Baer, R.. Bankier. A.T.. Biggin, M.D., Deininger. P.L.. Farrell. P.J Gibson. T.J . Hatfull, G., Hudson, G.S., Satchwell, S.C., Seguin. C., Tufnell, P.S. und Bareil, B.G. (1984) ONA sequence and expression of the B95-8 Epstein-Barr virus genome. *Nature (London)*, **310**, 207-211.
Cherepanov, P.P. und Wackernagel, W. (1995) Gene disruption in Escherichia coli: TcR and KmR cassettes with the option of Flp-catalyzed excision of the antibiotic-resistance determinant. Gene, **158**, 9-14.
Cohen, J.I., Wang, F., Mannick, J. und Kieff, E. (1989) Epstein-Barr virus nuclear protein 2 is a key determinant of lymphocyte transformation. *Proc. Natl. Acad. Sci. U S A*, **86**, 9558-9562.
Hammerschmidt. W. und Sugden, B. (1988) Identification and characterization of *oriLyt*, a lytic origin of DNA replication of Epstein-Barr virus. *Cell*, **55**, 427-433.
Hammerschmidt, W. und Sugden, B. (1989) Genetic analysis of immortalizing functions of Epstein-Barr virus in human B lymphocytes*. Nature (London)*, **340**. 393-397.
Hammerschmidt, W. und Sugden, W.M. (1993) US-A-5 194 601.
Hammerschmidt, W. und Sugden, B. (1995) EP-A- 0 694 613.
Hanahan, D. (1983) Studies on transformation of Escherichia coli with plasmids. *J. Mol. Biol*., **166**, 557-580.
Kempkes, B., Pich. D., Zeidler, R. und Hammerschmidt, W. (1995a) Immortalization of human primary B-lymphocytes *in vitro* with DNA. *Proc. Natl. Acad. Sci. USA.* **92**. 5875-5879.
Kempkes, B., Pich. D.. Zeidler, R., Sugden, B. und Hammerschmidt. W. (1995b) Immortalization of human B-lymphocytes by a plasmid containing 71 kpb of Epstein-Barr viral DNA. *J. Virol* **69**. 231-238
O'Connor. M., Peifer, M. und Bender, W. (1989) Construction of large DNA segments in Escherichia coli. *Science*, **244**, 1307-1312.
Yates. J L.. Warren, N. und Sugden. B. (1985) Stable replication of plasmids derived from Epstein-Barr virus in various mammalian cells. *Nature (London)*, **313**, 812-815.
Firth. N., Ippen-Ihler, K., und Skurray, R.A. (1996): Structure and function of the F factor and mechnism of conjugation. In Escherichia coli and Salmonella, F.C. Neidhardt, R. Curtiss III, J.L. Ingraham, E.C.C. lin, K.B. Low, B. Magasnik, W. S. Reznikoff. M. Riley. M. Schaechter and H.E. Umbarger, eds. (Washington, D.C.: American Society for Microbiology Press, 2377-2401).
Shizuya, H., Birren, B., Kim, U. J., Mancino, V., Slepak, T., Tachiiri, Y., und Simon, M. (1992): Cloning and stable maintenance of 300-kilobase-pair fragments of human DNA in Escherichia coli using an F-factor-based vector. Proc. Natl. Acad. Sci. USA 89, 8794-8797.

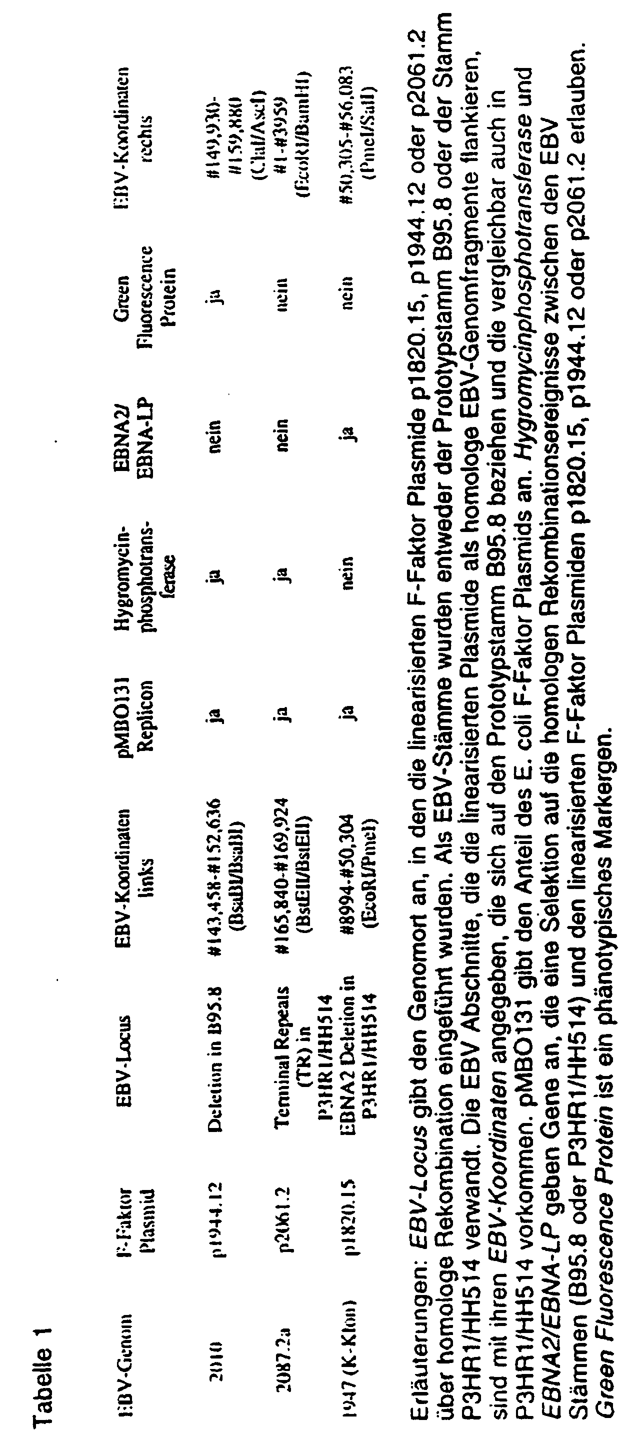

## Patentansprüche

1. Verfahren zur Herstellung von DNA-Virus-Vektoren, die sowohl in eukaryontischen als auch prokaryontischen Zellen replizierbar sind, umfassend zumindest die nachfolgenden Schritte:
(a) Einbringen eines DNA-Virus-Genoms ≥ 100 kbp in eine eukaryontische Zielzelle und Etablieren solcher Zellen, die das virale DNA-Genom zumindest in extrachromosomaler Form enthalten oder Einsetzen einer einer DNA-Virus-Genom ≥ 100 kbp in extrachromosomaler Form bereits natürlicherweise enthaltenden eukaryontischen Zielzelle;
(b) Einbringen eines DNA-Abschnitts in die eukaryontische Zielzelle, der zumindest, in funktioneller Anordnung, die Information für die Replikation des viralen DNA-Genoms in prokaryontischen Zellen, ein in eukaryontischen Zellen selektierbares Markergen und zumindest ein in prokaryontischen Zellen selektierbares Markergen umfaßt, und der von DNA-Abschnitten (homologe Abschnitte) aus dem DNA-Virus-Genom in einer eine Rekombination ermöglichenden Länge flankiert ist;
(c) Integration der unter (b) bezeichneten Abschnitte in das DNA-Virus-Genom durch Rekombination;
(d) Selektion auf diejenigen Zielzellen, die ein extrachromosomales rekombinantes virales DNA-Genom enthalten;
(e) wahlweise Aufreinigen und Isolieren des rekombinanten DNA-Virus-Vektor-Genoms.

2. Verfahren nach Anspruch 1, **dadurch gekennzeichnet, daß** das Genom des DNA-Virus ausgewählt wird aus einer Gruppe, umfassend eine Größe von ≥ 120 kbp, ≥ 150 kbp oder ≥ 170 kbp.

3. Verfahren nach Anspruch 1 oder 2, **dadurch gekennzeichnet, daß** das DNA-Virus zur Gruppe der Herpes-Viren, der Pox-Viren, Baculo-Viren oder der Iridio-Viren gehört.

4. Verfahren nach Anspruch 3, **dadurch gekennzeichnet, daß** das Herpes-Virus ein Alpha-Herpes-Virus, ein Beta-Herpes-Virus oder ein Gamma-Herpes-Virus ist.

5. Verfahren nach Anspruch 4, **dadurch gekennzeichnet, daß** das Alpha-Herpes-Virus ein Herpes-Simplex-Virus (HSV) oder ein Varicella-Zoster-Virus (VZV) ist, das Beta-Herpes-Virus ein Cytomegalie-Virus (CMV) ist und das Gamma-Herpes-Virus ein Epstein-Barr-Virus (EBV) ist.

6. Verfahren nach einem oder mehreren der vorhergehenden Ansprüche, **dadurch gekennzeichnet, daß** im Schritt (c) eine homologe Rekombination stattfindet.

7. Verfahren nach einem oder mehreren der vorhergehenden Ansprüche, **dadurch gekennzeichnet, daß** als DNA-Virus ein EBV-Virus verwendet wird.

8. Verfahren nach einem oder mehreren der vorhergehenden Ansprüche, **dadurch gekennzeichnet,**
**daß** das in eukaryontischen Zellen selektierbare Markergen ein Antibiotikumresistenzgen oder ein durch Fluoreszenz nachweisbares Protein ist.

9. Verfahren nach einem oder mehreren der vorhergehenden Ansprüche, **dadurch gekennzeichnet, daß** als die flankierenden homologen DNA-Virus-Sequenzen im Schritt (b) solche mit einer Größe ≥ 300 bp, bevorzugt ≥ 1 kbp, insbesondere bevorzugt ≥ 2 kbp eingesetzt werden.

10. Verfahren nach einem oder mehreren der vorhergehenden Ansprüche, **dadurch gekennzeichnet, daß** der DNA-Abschnitt im Schritt (b) vor dem Einbringen linearisiert wird.

11. Verfahren nach einem oder mehreren der vorhergehenden Ansprüche, **dadurch gekennzeichnet, daß** im Schritt (b) als DNA-Abschnitt zumindest ein Abschnitt des E. coli-Plasmids F-Faktor eingesetzt wird, der die Replikationsfunktion und einen für E. coli selektionierbaren Marker umfaßt.

12. Verfahren nach einem oder mehreren der vorhergehenden Ansprüche, **dadurch gekennzeichnet, daß** der DNA-Abschnitt im Schritt (b) zusätzlich die Information für zumindest ein therapeutisch wertvolles Gen aufweist.

13. Verfahren nach einem oder mehreren der vorhergehenden Ansprüche, **dadurch gekennzeichnet, daß** als Gen ein für ein Protein der Blutgerinnungskaskade kodierendes Gen, bevorzugt das Faktor VIII-Gen, ausgewählt wird.

14. Verfahren nach einem oder mehreren der vorhergehenden Ansprüche, **dadurch gekennzeichnet, daß** die homologen Abschnitte im Schritt (b).so ausgewählt werden, daß durch die Rekombination eine Punktmutation, mehre Nukleotide betreffende Mutation, Deletion, Addition oder Nukleotidaustausche in das virale DNA-Genom eingeführt wird.

15. Verfahren nach einem oder mehreren der vorhergehenden Ansprüche,
**dadurch gekennzeichnet,**
**daß** das isolierte, rekombinante DNA-Virus-Vektor-Genom in eine Prokaryontenzelle, bevorzugt eine E.coli-Zelle, eingeführt wird.

16. Verfahren nach einem oder mehreren der vorhergehenden Ansprüche,
**dadurch gekennzeichnet,**
**daß** das rekombinante DNA-Virus-Vektor-Genom in eine Eukaryontenzelle zur Expression von Fremdproteinen zumindest von einem therapeutisch wertvollen Gen eingeführt wird.

17. Verfahren nach einem oder mehreren der vorhergehenden Ansprüche,
**dadurch gekennzeichnet,**
**daß** das erhaltene rekombinante DNA-Virus-Vektor-Genom in eine andere Eukaryontenzelle als die in Anspruch 1 eingesetzte Zielzelle oder in eine Prokaryontenzelle eingeführt wird und in weiteren Verfahrensschritten Mutationen in das Rekombinante Genom eingeführt werden.

18. Verfahren nach Anspruch 14 oder 17,
**dadurch gekennzeichnet,**
**daß** die Mutationen Punktmutationen, mehrere Nukleotide betreffende Mutationen, Deletionen, Additionen oder Nukleotidaustausche umfassen.

19. Verfahren nach Anspruch 17, **dadurch gekennzeichnet, daß** die Mutationen durch Rekombination mit einem in der Prokaryontenzelle replizierbaren Vektor eingeführt werden.

20. Verfahren nach einem oder mehreren der Ansprüche 17, 18 oder 19, **dadurch gekennzeichnet, daß** das Einführen von Nukleotidadditionen das Einführen zumindest eines therapeutical wertvollen Genes umfaßt.

21. Verfahren nach einem oder mehreren der Ansprüche 14, 17, 18 oder 19, **dadurch gekennzeichnet, daß** eine Mutation auf einem oder mehreren der cis-aktiven Abschnitte des viralen DNA-Genoms eingeführt wird, die die Verpackung des viralen DNA-Genoms verhindert.

22. Verfahren nach einem oder mehreren der vorhergehenden Ansprüche, **dadurch gekennzeichnet, daß** im Schritt (a) das Einbringen des DNA-Virus-Genoms oder im Schritt (b) das Einbringen des DNA-Abschnitts durch Infektion, Transfektion oder Elektroporation erfolgt.

23. Verfahren nach einem oder mehreren der vorhergehenden Ansprüche,
**dadurch gekennzeichnet, daß**
die Prokaryontenanteile des DNA-Abschnitts im Schritt (b) so gewählt werden, daß sie teilweise zueinander homologe Sequenzen in einer solchen Länge besitzen, die zu einer homologen Rekombination befähigt sind, so daß nach einer homologen Rekombination die Prokaryontenanteile des DNA-Abschnitts aus (b) eliminiert werden.

24. Verfahren nach einem oder mehreren der vorhergehenden Ansprüche, **gekennzeichnet durch** die nachfolgenden weiteren Verfahrensschritte:
(f) Einführen des rekombinanten DNA-Virus-Vektor-Genoms in eine Prokaryontenzelle;
(g) Einführen eines Plasmids in die Prokaryontenzelle, das flankiert von DNA-Abschnitten in einer solchen Länge, die eine Rekombination mit dem DNA-Virus-Vektor-Genom ermöglichen, zumindest, in funktioneller Anordnung, eine DNA-Sequenz, die im Vergleich zu der auf dem DNA-Virus-Vektor-Genom vorliegenden homologen Sequenz eine Mutation enthält, und ein selektierbares Markergen enthält;
(h) Integration der unter (g) beschriebenen DNA-Sequenz und des Markergens **durch** Rekombination in das DNA-Virus-Genom;
(i) wahlweise Aufreinigen und Isolieren des rekombinanten DNA-Vektors.

25. Verfahren nach Anspruch 24,
**dadurch gekennzeichnet,**
**daß** als Prokaryontenzelle E. coli eingesetzt wird.

26. Verfahren nach Anspruch 24,
**dadurch gekennzeichnet,**
**daß** das Plasmid im Schritt (g) weiterhin von einer Rekombinase erkennbare Sequenzmotive enthält und vor dem Schritt (i) das durch die Rekombination eingeführte Markergen durch ortsspezifische Rekombination über die von der Rekombinase erkannten Sequenzmotive wieder entfernt wird.

## Claims

1. Method for the preparation of DNA virus vectors capable of replication in eukaryotic as well as in prokaryotic cells, at least comprising the following steps of:
(a) Introducing a DNA virus genome ≥ 100 kbp into an eukaryotic target cell and establishing such cells containing the viral DNA genome at least in extrachromosomal form or employing an eucaryotic target cell which already naturally contains a DNA virus genome ≥ 100 kbp in an extrachromosomal form;
b) introducing a DNA segment into the eukaryotic target cell at least comprising, operably linked to each other, the information for replication of the viral DNA genome in prokaryotic cells, a marker gene selectable in eucaryotic cells and at least one marker gene selectable in prokaryotic cells, and which is flanked by DNA segments (homologous segments) of the DNA virus genome having a length which enables recombination;
c) integrating the segments defined in (b) into the DNA virus genome by recombination;
d) selecting such target cells containing an extrachromosomal recombinant viral DNA genome;
e) optionally purifying and isolating the recombinant DNA virus vector genome.

2. Method according to claim 1 **characterized in that** said genome of the DNA virus is selected from a group comprising a size of ≥ 120 kbp, ≥ 150 kbp or ≥ 170 kbp.

3. Method according to claim 1 or 2 **characterized in that** said DNA virus belongs to the group of herpes viruses, pox viruses, baculoviruses, or iridioviruses.

4. Method according to claim 3 **characterized in that** said herpes virus is an alpha herpes virus, a beta herpes virus, or a gamma herpes virus.

5. Method according to claim 4 **characterized in that** said alpha herpes virus is a herpes simplex virus (HSV) or a varicella zoster virus (VZV), said beta herpes virus is a cytomegalovirus (CMV), and said gamma herpes virus is an Epstein-Barr virus.

6. Method according to one or more of the preceding claims **characterized in that** in said step
(c) a homologous recombination occurs.

7. Method according to one or more of the preceding claims **characterized in that** an EBV virus is used as said DNA virus.

8. Method according to one or more of the preceding claims **characterized in that** said marker gene selectable in eucaryotic cells is an antibiotic resistance gene or a protein detectable by fluorescence.

9. Method according to one or more of the preceding claims **characterized in that** as the flanking homologous DNA virus sequences in step (b) those are employed having a size of ≥ 300 bp, preferably ≥ 1 kbp, especially preferred ≥ 2 kbp.

10. Method according to one or more of the preceding claims **characterized in that** said DNA segment in step (b) is linearized prior to introduction.

11. Method according to one or more of the preceding claims **characterized in that** in said step (b) as the DNA segment is employed at least one segment of the E. coli plasmid F factor comprising the function for replication and a marker selectable in E. coli.

12. Method according to one or more of the preceding claims **characterized in that** said DNA segment in step (b) additionally has the information for at least one therapeutically valuable gene.

13. Method according to one or more of the preceding claims **characterized in that** a gene encoding a protein of the blood coagulation cascade, preferably the factor VIII gene, is selected as the gene.

14. Method according to one or more of the preceding claims **characterized in that** the homologous segments in step (b) are selected so that by recombination a point mutation, a mutation affecting several nucleic acids, deletion, addition or nucleotide substitutions is introduced into the viral DNA-genome.

15. Method according to one or more of the preceding claims **characterized in that** the isolated recombinant DNA virus vector genome is introduced into a prokaryotic cell, preferably an E. coli cell.

16. Method according to one or more of the preceding claims **characterized in that** the recombinant DNA virus vector genome is introduced into an eukaryotic cell for expression of foreign proteins from at least one therapeutically valuable gene.

17. Method according to one or more of the preceding claims **characterized in that** the resulting recombinant DNA virus vector genome is introduced in a different eucaryotic cell than the target cell employed in claim 1 or in a procaryotic cell and in further process steps mutations are introduced in the recombinant genome.

18. Method according to claim 14 or 17 **characterized in that** said mutations comprise point mutations, mutations affecting several nucleotides, deletions, additions, or nucleotide substitutions.

19. Method according to claim 17 **characterized in that** the mutations are introduced by recombination with a vector capable of replication in the prokaryotic cell.

20. Method according to one or more of claims 17, 18, or 19 **characterized in that** the introduction of nucleotide additions comprises the introduction of at least one therapeutically valuable gene.

21. Method according to one or more of claims 14, 17, 18, or 19 **characterized in that** a mutation is introduced on one or more of the cis-acting segments of the viral DNA genome which prevents the packaging of the viral DNA genome.

22. Method according to one or more of the preceding claims **characterized in that** in said step (a) the introduction of the DNA virus genome or in said step (b) the introduction of the DNA segment is performed by infection, transfection or electroporation.

23. Method according to one or more of the preceding claims **characterized in that** the prokaryotic portions of the DNA segment in step (b) are selected so that they have sequences which are in part homologous to each other and have a length rendering them capable for homologous recombination so that following homologous recombination the prokaryotic portions of the DNA segment in (b) are eliminated.

24. Method according to one or more of the preceding claims **characterized by** the following further process steps of:
(f) Introducing a recombinant DNA virus vector genome into a prokaryotic cell;
(g) introducing a plasmid into the prokaryotic cell containing flanked by DNA segments of a length sufficient to enable a recombination with the DNA virus vector genome at least, operably linked to each other, a DNA sequence which bears a mutation compared to the homologous sequence present on the DNA virus vector genome and a selectable marker gene;
(h) integrating the sequence and the marker gene described in (g) by recombination into the DNA virus genome;
(i) optionally purifying and isolating the recombinant DNA vector.

25. Method according to claim 24 **characterized in that** E. coli is used as a procaryotic cell.

26. Method according to claim 24 **characterized in that** the plasmid in step (g) further contains sequence motifs recognizable by a recombinase and that prior to step (i) the marker gene introduced by the recombination is removed by site-directed recombination via the sequence motifs recognized by the recombinase.

## Revendications

1. Procédé de préparation de vecteurs viraux à ADN qui sont réplicables aussi bien dans des cellules eucaryotes que dans des cellules procaryotes, comprenant au moins les étapes suivantes :
(a) Insertion d'un génome de virus à ADN ≥ 100 kpb dans une cellule cible eucaryote et établissement de ces cellules contenant le génome du virus à ADN au moins sous forme extrachromosomique ou utilisation d'une cellule cible eucaryote contenant déjà naturellement un génome de virus à ADN ≥ 100 kpb sous forme extrachromosomique ;
(b) Insertion dans la cellule cible eucaryote d'un segment d'ADN qui comprend au moins, selon un agencement fonctionnel, l'information nécessaire à la réplication du génome du virus à ADN dans des cellules procaryotes, un gène de marquage sélectionnable dans des cellules eucaryotes et au moins un gène de marquage sélectionnable dans des cellules procaryotes et qui est flanqué par des segments d'ADN (segments homologues) issus du génome du virus à ADN en une longueur permettant une recombinaison ;
(c) Intégration des fragments décrits en (b) dans le génome du virus à ADN par recombinaison ;
(d) Criblage des cellules cibles qui contiennent un génome du virus à ADN recombinant extrachromosomique ;
(e) Le cas échéant, purification et isolement du génome du virus à ADN recombinant extrachromosomique.

2. Procédé selon la revendication 1, **caractérisé en ce que** le génome du virus à ADN est choisi dans un groupe constitué par une taille ≥ 120 kpb, ≥ 150 kpb ou ≥ 170 kpb.

3. Procédé selon la revendication 1 ou la revendication 2, **caractérisé en ce que** le virus à ADN appartient au groupe des herpèsvirus, des poxvirus, des baculovirus ou des iridiovirus.

4. Procédé selon la revendication 3, **caractérisé en ce que** l'herpèsvirus est un herpèsvirus alpha, un herpèsvirus bêta ou un herpèsvirus gamma.

5. Procédé selon la revendication 4, **caractérisé en ce que** l'herpèsvirus alpha est un virus herpès simplex (HSV) ou un virus varicelle-zona (VZV), **en ce que** l'herpèsvirus bêta est un cytomégalovirus (CMV) et **en ce que** l'herpès virus gamma est un virus Epstein-Barr (EBV)

6. Procédé selon l'une ou plusieurs des revendications précédentes, **caractérisé en ce que**, à l'étape (c), une recombinaison homologue a lieu.

7. Procédé selon l'une ou plusieurs des revendications précédentes, **caractérisé en ce que** le virus à ADN utilisé est un virus EBV.

8. Procédé selon l'une ou plusieurs des revendications précédentes, **caractérisé en ce que** le gène de marquage sélectionnable dans des cellules eucaryotes est un gène de résistance à un antibiotique ou une protéine détectable par fluorescence.

9. Procédé selon l'une ou plusieurs des revendications précédentes, **caractérisé en ce que**, à l'étape (b), on utilise comme séquences homologues adjacentes du virus à ADN des séquences d'une taille ≥ 300 pb, de préférence ≥ 1 kpb et de façon particulièrement préférée ≥ 2 kpb.

10. Procédé selon l'une ou plusieurs des revendications précédentes, **caractérisé en ce que**, à l'étape (b), le segment d'ADN est linéarisé avant son insertion.

11. Procédé selon l'une ou plusieurs des revendications précédentes, **caractérisé en ce que**, à l'étape (b), on utilise comme fragment d'ADN au moins un segment du facteur F de plasmide de *E. coli* qui comprend la fonction de réplication et un marqueur sélectionnable pour *E. coli*.

12. Procédé selon l'une ou plusieurs des revendications précédentes, **caractérisé en ce que**, à l'étape (b), le fragment d'ADN présente en plus l'information pour au moins un gène thérapeutiquement utile.

13. Procédé selon l'une ou plusieurs des revendications précédentes, **caractérisé en ce que** le gène choisi est un gène codant pour une protéine de la cascade de la coagulation sanguine, de préférence le gène du facteur VIII.

14. Procédé selon l'une ou plusieurs des revendications précédentes, **caractérisé en ce que**, à l'étape (b), les fragments homologues sont choisis de façon à introduire dans le génome de l'ADN viral, par la recombinaison, une mutation ponctuelle, une mutation concernant plusieurs nucléotides, une délétion, une addition ou des permutations de nucléotides.

15. Procédé selon l'une ou plusieurs des revendications précédentes, **caractérisé en ce que** le génome vecteur viral à ADN recombinant isolé est introduit dans une cellule procaryote, de préférence une cellule de *E. coli*.

16. Procédé selon l'une ou plusieurs des revendications précédentes, **caractérisé en ce que** le génome vecteur viral à ADN recombinant isolé est introduit dans une cellule eucaryote pour l'expression de protéines étrangères d'au moins un gène thérapeutiquement utile.

17. Procédé selon l'une ou plusieurs des revendications précédentes, **caractérisé en ce que** le génome vecteur viral à ADN recombinant ainsi obtenu est introduit dans une cellule eucaryote autre que la cellule cible utilisée dans la revendication 1 ou dans une cellule procaryote et **en ce que** des mutations sont introduites dans le génome recombinant dans d'autres étapes de procédé.

18. Procédé selon la revendication 14 ou la revendication 17, **caractérisé en ce que** les mutations comprennent des mutations ponctuelles, des mutations concernant plusieurs nucléotides, des délétions, des additions ou des permutations de nucléotides.

19. Procédé selon la revendication 17, **caractérisé en ce que** les mutations sont introduites par recombinaison avec une vecteur réplicable dans la cellule procaryote.

20. Procédé selon l'une ou plusieurs des revendications 17, 18 ou 19, **caractérisé en ce que** l'introduction d'additions de nucléotides comprend l'introduction d'au moins un gène thérapeutiquement utile.

21. Procédé selon l'une ou plusieurs des revendications 14, 17, 18 ou 19, **caractérisé en ce que** l'on introduit sur un ou plusieurs des segments cis-actifs du génome ADN viral une mutation qui empêche l'empaquetage du génome ADN viral.

22. Procédé selon l'une ou plusieurs des revendications précédentes, **caractérisé en ce que** l'introduction du génome viral à ADN à l'étape (a) ou l'introduction du fragment d'ADN à l'étape (b) se fait par infection, transfection ou électroporation.

23. Procédé selon l'une ou plusieurs des revendications précédentes, **caractérisé en ce que** les parties procaryotes du fragment d'ADN à l'étape (b) sont choisies de façon à ce qu'elles possèdent des séquences partiellement homologues les unes des autres, aptes à une recombinaison homologue, avec une longueur telle que, après une recombinaison homologue, les parties procaryotes du fragment d'ADN de l'étape (b) sont éliminées.

24. Procédé selon l'une ou plusieurs des revendications précédentes, **caractérisé par** les étapes de procédé supplémentaires suivantes :
(f) Introduction du génome de vecteur viral à ADN dans une cellule procaryote ;
(g) Introduction dans la cellule procaryote d'un plasmide contenant au moins, flanquée de fragments d'ADN d'une longueur permettant une recombinaison avec le génome de vecteur viral à ADN et selon un arrangement fonctionnel, une séquence d'ADN qui contient une mutation par rapport à la séquence présente dans le génome de vecteur viral à ADN et un gène de marquage sélectionnable ;
(h) Intégration de la séquence d'ADN décrite en (g) et du gène de marquage par recombinaison dans le génome de virus à ADN ;
(i) Le cas échéant, purification et isolation du vecteur d'ADN recombinant.

25. Procédé selon la revendication 24, **caractérisé en ce que** la cellule procaryote utilisée est une cellule de *E. coli.*

26. Procédé selon la revendication 24, **caractérisé en ce que** le plasmide de l'étape (g) contient en plus des motifs de séquence reconnaissables par une recombinase et **en ce que**, avant l'étape (i), le gène de marquage introduit par la recombinaison est à nouveau éliminé par une recombinaison dirigée sur un site par l'intermédiaire des motifs de séquence reconnus par la recombinase.
